(19) Europäisches Patentamt / European Patent Office / Office européen des brevets

(11) **EP 3 967 398 A1**

(12) **EUROPEAN PATENT APPLICATION**
published in accordance with Art. 153(4) EPC

(43) Date of publication:
**16.03.2022 Bulletin 2022/11**

(21) Application number: **21738836.2**

(22) Date of filing: **08.01.2021**

(51) International Patent Classification (IPC):
**B01J 23/883** (2006.01)   **C07B 61/00** (2006.01)
**C07C 27/00** (2006.01)   **C07C 47/22** (2006.01)
**C07C 57/05** (2006.01)

(52) Cooperative Patent Classification (CPC):
**B01J 23/883; C07B 61/00; C07C 27/00;
C07C 47/22; C07C 57/04**

(86) International application number:
**PCT/JP2021/000588**

(87) International publication number:
**WO 2021/141134 (15.07.2021 Gazette 2021/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.01.2020   JP 2020002508**

(71) Applicant: **Nippon Kayaku Kabushiki Kaisha
Tokyo 100-0005 (JP)**

(72) Inventors:
• **YASUDA, Shogo**
  **Sanyoonoda-shi, Yamaguchi 757-8686 (JP)**
• **OKUMURA, Shigeki**
  **Sanyoonoda-shi, Yamaguchi 757-8686 (JP)**
• **HIRAOKA, Ryota**
  **Sanyoonoda-shi, Yamaguchi 757-8686 (JP)**
• **FUKUNAGA, Seiichiro**
  **Sanyoonoda-shi, Yamaguchi 757-8686 (JP)**

(74) Representative: **Gille Hrabal
Partnerschaftsgesellschaft mbB
Patentanwälte
Brucknerstraße 20
40593 Düsseldorf (DE)**

(54) **CATALYST, METHOD FOR PRODUCING COMPOUND USING SAME, AND COMPOUND**

(57)    A catalyst containing, as an essential component, molybdenum; bismuth; and cobalt, in which, with respect to a peak intensity at $2\theta = 25.3° \pm 0.2°$ in an X-ray diffraction pattern obtained by using CuKa rays as an X-ray source, a changing rate (Q1) per 1000 hours of reaction time represented by the following formulae (1) to (4) is 16 or less.

$$Q1 = \{(U1/F1 - 1) \times 100\}/T \times 1000 \quad (1)$$

$$F1 = \text{(peak intensity of catalyst before oxidation reaction at } 2\theta = 25.3° \pm 0.2°)/\text{(peak intensity of catalyst before oxidation reaction at } 2\theta = 26.5° \pm 0.2°) \times 100 \quad (2)$$

$$U1 = \text{(peak intensity of catalyst after oxidation reaction at } 2\theta = 25.3° \pm 0.2°)/\text{(peak intensity of catalyst after oxidation reaction at } 2\theta = 26.5° \pm 0.2°) \times 100 \quad (3)$$

$$T = \text{time (hr) during which oxidation reaction is carried out} \quad (4)$$

FIG. 1

**Description**

TECHNICAL FIELD

The present invention relates to a catalyst, a method for producing a compound using the same, and a compound.

BACKGROUND ART

A method for using propylene, isobutylene, t-butyl alcohol or the like as a raw material to produce a corresponding unsaturated aldehyde or unsaturated carboxylic acid, or a catalytic gas phase oxidation for producing 1,3-butadiene from butenes are widely carried out industrially.

In particular, regarding the method for using propylene, isobutylene, t-butyl alcohol or the like as a raw material to produce a corresponding unsaturated aldehyde or unsaturated carboxylic acid, many reports have been made for means for improving the yield and improving the catalytic activity (for example, Patent Literature 1, etc.).

Among them, Patent Literature 2 describes that a catalyst exhibiting a high yield and a high selectivity can be obtained by controlling a ratio Ri = Pi/Ph of an intensity Pi of a diffraction peak (i) of $\beta$-$Bi_2Mo_2O_9$ appearing at $2\theta = 27.76°$ $\pm$ 0.1° to an intensity Ph of a diffraction peak (h) of $CoMoO_4$ appearing at $2\theta = 26.5°$ $\pm$ 0.1° in an X-ray diffraction pattern of a catalytically active component using Cu-K$\alpha$ rays, to be in a range of 0.4 or more and 2.0 or less.

Patent Literature 3 describes that catalyst performance such as catalytic activity and selectivity can be improved by controlling crystallinity T in a range of $2\theta = 5°$ or more and 90° or less to be in a range of 4% or more and 18% or less, as measured by X-ray diffraction analysis of a catalytically active component using Cu-K$\alpha$ rays.

Further, Patent Literature 4 describes that, in an oxide catalyst in which a ratio Ri = Pa/Pc of an intensity Pa of a diffraction peak (a) of a $Bi_{10}Mo_3O_{24}$ phase appearing at $2\theta = 27.4°$ $\pm$ 0.2° to an intensity Pc of a diffraction peak (c) of a $CoMoO_4$ phase appearing at a position of $2\theta = 26.4°$ $\pm$ 0.2° is $0.2 \leq Ri \leq 1.0$, a time-course increase in the activity in a reaction is small, and an unsaturated aldehyde can be generated at a high yield.

On the other hand, further improvement in yield and improvement in catalytic activity are required for subjecting propylene, isobutylene, t-butyl alcohol or butene to a partial oxidation reaction to produce the corresponding unsaturated aldehyde and/or unsaturated carboxylic acid and required for producing a conjugated diene by oxidative dehydrogenation of butenes. For example, the yield of a target product influences an amount of propylene, isobutylene, t-butyl alcohol, or the like required for the production and greatly influences the production cost. The catalytic activity influences a salt bath temperature (reaction temperature) when the target product is produced, and when a catalyst having low activity is used, the salt bath temperature must be increased in order to maintain the yield of the target product. Then, the catalyst is subjected to thermal stress, and the selectivity and the yield are reduced, and thus this may lead to a reduction in the life of the catalyst.

In particular, regarding the life of the catalyst, a relationship between the physical properties of the catalyst or the time-course change of the physical properties and the life of the catalyst has been unclear. When an unsaturated aldehyde, an unsaturated carboxylic acid, or a conjugated diene is produced using a catalyst with a high yield and/or a high selectivity kept, it has not been clear what kind of characteristics the catalyst should be used and what index should be used for management.

CITATION LIST

PATENT LITERATURE

Patent Literature 1: WO 2016/136882
Patent Literature 2: JP-A-2017-024009
Patent Literature 3: WO 2010/038677
Patent Literature 4: JP-A-2018-140326

SUMMARY OF INVENTION

TECHNICAL PROBLEM

The present invention proposes a catalyst which is used in a method for using propylene, isobutylene, t-butyl alcohol, or the like as a raw material to produce a corresponding unsaturated aldehyde or unsaturated carboxylic acid, or used in a catalytic gas phase oxidation method for producing 1,3-butadiene from butenes, and which has high catalytic activity and high selectivity of a target product. The use of the catalyst of the present invention allows for performing long-term operation of the catalytic gas phase oxidation method safely, stably, and at low cost.

SOLUTION TO PROBLEM

The problem of deterioration in selectivity at the time of reaction often occurs depending on a type of the catalyst in related art, and the reason is unknown. However, the present invention has been achieved by extracting and evaluating the catalyst after the reaction. That is, the present inventors have found for the first time that the characteristic that a changing rate of a peak intensity at $2\theta = 25.3° \pm 0.2°$ after the reaction is small leads to prevention of the deterioration in selectivity.

That is, the present invention relates to the following 1) to 11).

1) A catalyst, containing, as an essential component,

molybdenum;
bismuth; and cobalt, wherein
with respect to a peak intensity at $2\theta = 25.3° \pm 0.2°$ in an X-ray diffraction pattern obtained by using CuKa rays as an X-ray source, a changing rate (Q1) per 1000 hours of reaction time represented by the following formulae (1) to (4) is 16 or less.

$$Q1 = \{(U1/F1 - 1) \times 100\}/T \times 1000 \quad (1)$$

$$F1 = (\text{peak intensity of catalyst before oxidation reaction at } 2\theta = 25.3° \pm 0.2°)/(\text{peak}$$
$$\text{intensity of catalyst before oxidation reaction at } 2\theta = 26.5° \pm 0.2°) \times 100 \quad (2)$$

$$U1 = (\text{peak intensity of catalyst after oxidation reaction at } 2\theta = 25.3° \pm 0.2°)/(\text{peak}$$
$$\text{intensity of catalyst after oxidation reaction at } 2\theta = 26.5° \pm 0.2°) \times 100 \quad (3)$$

$$T = \text{time (hr) during which oxidation reaction is carried out} \quad (4)$$

2) The catalyst according to 1), wherein, with respect to a peak intensity at $2\theta = 25.3° \pm 0.2°$ in an X-ray diffraction pattern obtained by using CuKa rays as an X-ray source, a changing amount (D1) per 1000 hours of reaction time represented by the following formula (5) and the formulae (2) to (4) is 4.1 or less.

$$D1 = (U1 - F1)/T \times 1000 \quad (5)$$

3) The catalyst according to 1) or 2), wherein a composition of a catalytically active component is represented by the following formula (A):

$$Mo_{a1}Bi_{b1}Ni_{c1}Co_{d1}Fe_{e1}X_{f1}Y_{g1}Z_{h1}O_{i1} \quad (A)$$

(in the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silica, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, h1, and i1 represent the number of atoms of molybdenum, bismuth, nickel, cobalt, iron, X, Y, Z, and oxygen, respectively; when a1 = 12, $0 < b1 \leq 7$, $0 \leq c1 \leq 10$, $0 < d1 \leq 10$, $0 < c1 + d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element).

4) The catalyst according to any one of 1) to 3), wherein a catalytically active component is carried on an inert carrier in the catalyst.

5) The catalyst according to 4), wherein the inert carrier is silica, alumina, or a mixture thereof.

6) The catalyst according to any one of 1) to 5), which is a catalyst for producing at least one of an unsaturated aldehyde compound, an unsaturated carboxylic acid compound, and a conjugated diene.

7) A method for producing at least one of an unsaturated aldehyde compound, an unsaturated carboxylic acid compound, and a conjugated diene using the catalyst according to any one of 1) to 6).

8) The production method according to 7), wherein the unsaturated aldehyde compound is acrolein, the unsaturated carboxylic acid compound is acrylic acid, and the conjugated diene is 1,3-butadiene.

9) An unsaturated aldehyde compound, an unsaturated carboxylic acid compound, or a conjugated diene produced using the catalyst according to any one of 1) to 6).

10) A method for producing at least one of an unsaturated aldehyde compound, an unsaturated carboxylic acid compound, and a conjugated diene using a catalyst containing molybdenum, bismuth, and cobalt as an essential component, wherein, with respect to a peak intensity of the catalyst at $2\theta = 25.3° \pm 0.2°$ in an X-ray diffraction pattern obtained by using CuKa rays as an X-ray source, a changing rate (Q4) per 1000 hours of reaction time represented by the following formulae (14) to (17) is 16 or less.

$$Q4 = \{(U4/F4 - 1) \times 100\}/T \times 1000 \quad (14)$$

$$U4 = (\text{peak intensity of catalyst after oxidation reaction at } 2\theta = 25.3° \pm 0.2°)/(\text{peak intensity of catalyst after oxidation reaction at } 2\theta = 26.5° \pm 0.2°) \times 100 \quad (15)$$

$$F4 = (\text{peak intensity of catalyst before oxidation reaction at } 2\theta = 25.3° \pm 0.2°)/(\text{peak intensity of catalyst before oxidation reaction at } 2\theta = 26.5° \pm 0.2°) \times 100 \quad (16)$$

$$T = \text{time (hr) during which oxidation reaction is carried out} \quad (17)$$

11) The method for producing at least one of an unsaturated aldehyde compound, an unsaturated carboxylic acid compound, and a conjugated diene according to 10), wherein a changing amount (D4) per 1000 hours of the reaction time represented by the following formula (18) is 4.1 or less.

$$D4 = (U4 - F4)/T \times 1000 \quad (18)$$

ADVANTAGEOUS EFFECTS OF INVENTION

The catalyst of the present invention allows for maintaining a high selectivity in a catalytic gas phase oxidation or a catalytic gas phase oxidation dehydrogenation, and is effective in improving yield. In particular, the catalyst is useful in the case of using propylene, isobutylene, t-butyl alcohol, or the like as a raw material to produce a corresponding unsaturated aldehyde or unsaturated carboxylic acid.

BRIEF DESCRIPTION OF DRAWINGS

FIG. 1 is a diagram illustrating an X-ray diffraction pattern of a catalyst (catalyst 3-1) in Example 3.
FIG. 2 is a diagram illustrating an X-ray diffraction pattern of a catalyst (catalyst 3-2) in Example 3.

FIG. 3 is a diagram illustrating an X-ray diffraction pattern of a catalyst (catalyst 4-1) in Comparative Example 1.
FIG. 4 is a diagram illustrating an X-ray diffraction pattern of a catalyst (catalyst 4-2) in Comparative Example 1.

DESCRIPTION OF EMBODIMENTS

Hereinafter, embodiments of the present invention will be described. In the present specification, a catalytic gas phase oxidation and a catalytic gas phase oxidation dehydrogenation may be collectively referred to simply as an oxidation reaction.

[Changing Rate (Q1) of Peak Intensity at $2\theta = 25.3° \pm 0.2°$ Per 1000 Hours of Reaction Time in X-Ray Diffraction Pattern]

A catalyst of the present embodiment is characterized in a changing rate (Q1) per 1000 hours of reaction time expressed by the following formulae (1) to (4) with respect to a peak intensity at $2\theta = 25.3° \pm 0.2°$ in the X-ray diffraction pattern obtained using CuKa rays as an X-ray source.

$$Q1 = \{(U1/F1 - 1) \times 100\}/T \times 1000 \qquad (1)$$

$$F1 = \text{(peak intensity of catalyst before oxidation reaction at } 2\theta = 25.3° \pm 0.2°)/\text{(peak intensity of catalyst before oxidation reaction at } 2\theta = 26.5° \pm 0.2°) \times 100 \qquad (2)$$

$$U1 = \text{(peak intensity of catalyst after oxidation reaction at } 2\theta = 25.3° \pm 0.2°)/\text{(peak intensity of catalyst after oxidation reaction at } 2\theta = 26.5° \pm 0.2°) \times 100 \qquad (3)$$

$$T = \text{time (hr) during which oxidation reaction is carried out} \qquad (4)$$

The peak intensity (F1) and the peak intensity (UI) are determined by normalization based on the peak intensity observed at $2\theta = 26.5° \pm 0.2°$, but in the changing rate (Q1), U1 is divided by F1, and thus the changing rate (Q1) is calculated substantially based on a value determined by (peak intensity of catalyst after oxidation reaction at $2\theta = 25.3°$ $\pm 0.2°$) / (peak intensity of catalyst before oxidation reaction at $2\theta = 25.3° \pm 0.2°$).

Here, the peak intensity will be described. The peak intensity at $2\theta = 25.3° \pm 0.2°$ means a local maximum value of a signal observed in a range of $2\theta = 25.3° \pm 0.2°$, and the nature of the peak intensity indicates a peak height of a crystal phase of $\alpha$-CoMoO$_4$. The peak intensity at $2\theta = 26.5° \pm 0.2°$ means a maximum value of a signal observed in a range of $2\theta = 26.5° \pm 0.2°$, and the nature of the peak intensity indicates a peak height of a crystal phase of $\beta$-CoMoO$_4$. That is, the present invention is based on the finding that when the changing rate (Q1) of the peak intensity of the crystal phase of $\alpha$-CoMoO$_4$ observed at $2\theta = 25.3° \pm 0.2°$ per 1000 hours of reaction time due to the oxidation reaction with respect to the peak intensity of the crystal phase of $\beta$-CoMoO$_4$ observed at $2\theta = 26.5° \pm 0.2°$ is equal to or less than a certain value, specifically, is 16 or less, the high selectivity is stably maintained.

As will be described later, the crystal phase of $\alpha$-CoMoO$_4$ changes and the stability thereof also changes depending on the composition and production method of the catalyst. This can be confirmed particularly by focusing on the changing rate (Q1) of the peak intensity at $2\theta = 25.3° \pm 0.2°$ per 1000 hours of reaction time. The range of Q1 is 16 or less as described above, and an upper limit of the range is more preferably 15, 10, 7.0, 5.0, 2.2, 2.0, 1.5, 1.2, 1.0, 0.70, and 0.50 in order, more preferably 0.0, still more preferably -5.0, and most preferably -7.0. A lower limit of the range may not be set, but is preferably -100, -80, -60, -40, and -20 in order, more preferably -15, still more preferably -10, and most preferably -8.0. That is, a more preferred range of the changing rate (Q1) of the peak intensity per 1000 hours of reaction time is set by the upper and lower limits described above, and is, for example, -40 or more and 15 or less, and most preferably -8.0 or more and -7.0 or less.

Examples of a method of measuring an X-ray diffraction angle ($2\theta$) of the catalyst include measuring the X-ray diffraction angle ($2\theta$) under conditions of X-ray CuKa rays ($\lambda = 0.154$ nm), an output of 40 kV, 30 mA, a measurement range of 10° to 60°, and a measurement speed of 10° per minute by using Ultima IV manufactured by Rigaku Corporation, but the method is not limited thereto as long as the method does not depart from the measurement principle. In addition, for the peak intensity of the present embodiment, the calculation is performed after the background and halo pattern have been eliminated as described in Patent Literature 3 in the X-ray diffraction pattern before the calculation. In addition, when each of the peaks does not have a clear local maximum value within the corresponding range of 2θ, or does not have a peak shape, or when it is not determined that the peak is a clear peak due to too much noise, the peak intensity is assumed to be 0 in the present embodiment.

[Changing Rate of Other Peaks in X-Ray Diffraction Pattern]

There are other X-ray diffraction peaks attributed to α-CoMoO$_4$. With respect to the peak intensity of the catalyst observed in the range of 2θ = 32.7° ± 0.2° in the X-ray diffraction pattern obtained by using CuKa rays as an X-ray source, it is preferable that a changing rate (Q2) per 1000 hours of the reaction time represented by the following formulae (6) to (8) and the above formula (4) is 4.8 or less. U2 is divided by F2 in the changing rate (Q2), and thus the changing rate (Q2) is calculated substantially based on a value obtained by (peak intensity of catalyst after oxidation reaction at 2θ = 32.7° ± 0.2°) ÷ (peak intensity of catalyst before oxidation reaction at 2θ = 32.7° ± 0.2°).

$$Q2 = \{(U2/F2 - 1) \times 100\}/T \times 1000 \quad (6)$$

$$F2 = (\text{peak intensity of catalyst before oxidation reaction at } 2\theta = 32.7° \pm 0.2°)/(\text{peak}$$
$$\text{intensity of catalyst before oxidation reaction at } 2\theta = 26.5° \pm 0.2°) \times 100 \quad (7)$$

$$U2 = (\text{peak intensity of catalyst after oxidation reaction at } 2\theta = 32.7° \pm 0.2°)/(\text{peak}$$
$$\text{intensity of catalyst after oxidation reaction at } 2\theta = 26.5° \pm 0.2°) \times 100 \quad (8)$$

An upper limit of the changing rate (Q2) of the peak intensity observed in the range of 2θ = 32.7° ± 0.2° per 1000 hours of the reaction time is preferably 4.0, 3.0, 2.7, 2.5, 2.0, 1.8, or 1.6 in order, more preferably 1.0, still more preferably 0.0, and most preferably -10. A lower limit of the changing rate may not be set, but is preferably -100, -80, -60, and -40 in order, more preferably -20, still more preferably -15, and most preferably -14. That is, a more preferred range of the changing rate (Q2) of the peak intensity is set by the upper and lower limits described above, and is, for example, -40 or more and 4.0 or less, and most preferably -14 or more and -10 or less.

In addition to the X-ray diffraction peak attributed to α-CoMoO$_4$, with respect to the peak intensity of the catalyst observed in the range of 2θ = 47.4° ± 0.2° in the X-ray diffraction pattern obtained by using CuKa ray as an X-ray source, it is preferable that a changing rate (Q3) per 1000 hours of the reaction time represented by the following formulae (9) to (11) and the above formula (4) is 8.7 or less. U3 is divided by F3 in a changing rate (Q3), and thus the changing rate (Q3) is calculated substantially based on a value obtained by (peak intensity of catalyst after oxidation reaction at 2θ = 47.4° ± 0.2°) ÷ (peak intensity of catalyst before oxidation reaction at 2θ = 47.4° ± 0.2°).

$$Q3 = \{(U3/F3 - 1) \times 100\}/T \times 1000 \quad (9)$$

$$F3 = (\text{peak intensity of catalyst before oxidation reaction at } 2\theta = 47.4° \pm 0.2°)/(\text{peak}$$
$$\text{intensity of catalyst before oxidation reaction at } 2\theta = 26.5° \pm 0.2°) \times 100 \quad (10)$$

$$U3 = (\text{peak intensity of catalyst after oxidation reaction at } 2\theta = 47.4° \pm 0.2°)/(\text{peak}$$
$$\text{intensity of catalyst after oxidation reaction at } 2\theta = 26.5° \pm 0.2°) \times 100 \quad (11)$$

An upper limit of the changing rate (Q3) of the peak intensity observed in the range of 2θ = 47.4° ± 0.2° per 1000 hours of the reaction time is preferably 8.0, 3.0, 2.2, 2.0, 1.7, or 1.5 in order, more preferably 1.0, still more preferably 0.5, and most preferably 0.3. A lower limit of the changing rate may not be set, but is preferably -100, -80, -60, -40, -20, or -10 in order, more preferably -5.0, still more preferably -1.0, and most preferably 0.0. That is, a more preferred range of the changing rate (Q3) of the peak intensity is set by the upper and lower limits described above, and is, for example, -40 or more and 8.0 or less, and most preferably 0.0 or more and 0.3 or less.

[Changing Amount (D1) of Peak Intensity at 2θ = 25.3° ± 0.2° Per 1000 Hours of Reaction Time in X-ray Diffraction Pattern]

In the catalyst of the present embodiment, with respect to a peak intensity at 2θ = 25.3° ± 0.2° in the X-ray diffraction pattern obtained by using CuKα rays as an X-ray source, a changing amount (D1) per 1000 hours of reaction time represented by the following formula (5) and the above formulae (2) to (4) is 4.1 or less.

$$D1 = (U1 - F1)/T \times 1000 \quad (5)$$

It has been found that when the changing amount (D1) of the peak intensity at 2θ = 25.3° ± 0.2° per 1000 hours of reaction time of the oxidation reaction with respect to the peak intensity at 2θ = 26.5° ± 0.2° is equal to or less than a certain value, specifically, is as small as 4.1 or less, the high selectivity is stably maintained. As will be described later, the crystal phase of α-CoMoO$_4$ changes and the stability thereof also changes depending on the composition and production method of the catalyst. This can be confirmed particularly by focusing on the changing amount (D1) of the peak intensity at 2θ = 25.3° ± 0.2° per 1000 hours of reaction time. The range of D1 is preferably 4.1 or less as described above, and an upper limit of the range is more preferably 3.0, 2.0, 1.9, 1.5, 1.0, 0.50, 0.30, 0.20, or 0.10 in order, still more preferably 0.0, yet still more preferably -1.0, and most preferably -1.3. A lower limit of the range may not be set, but is preferably -17, -15, or -10 in order, more preferably -5.0, still more preferably -2.0, and most preferably -1.5. That is, a more preferred range of the changing amount (D1) of the peak intensity per 1000 hours of the reaction time is set by the upper and lower limits described above, and is, for example, -10 or more and 3.0 or less, or the like, and most preferably -1.5 or more and -1.3 or less.

[Changing Amount of Other Peaks in X-ray Diffraction Pattern]

There are other X-ray diffraction peaks attributed to α-CoMoO$_4$. With respect to the peak intensity of the catalyst observed in the range of 2θ = 32.7° ± 0.2° in the X-ray diffraction pattern obtained by using CuKα rays as an X-ray source, it is preferable that the changing amount (D2) of the peak intensity per 1000 hours of the reaction time represented by the following formula (12) and the above formulae (4), (7), and (8) is 0.80 or less.

$$D2 = (U2 - F2)/T \times 1000 \quad (12)$$

An upper limit of the changing amount (D2) of the peak intensity observed in the range of 2θ = 32.7° ± 0.2° per 1000 hours of the reaction time is preferably 0.50, 0.30, or 0.20 in order, more preferably 0.10, still more preferably 0.0, and most preferably -1.0. A lower limit of the changing amount may not be set, but is preferably -8.7, -8.0, or -5.0 in order, more preferably -3.0, still more preferably -2.0, and most preferably -1.2. That is, a more preferred range of the changing amount (D2) of the peak intensity is set by the upper and lower limits described above, and is, for example, -5.0 or more and 0.50 or less, or the like, and most preferably -1.2 or more and -1.0 or less.

In addition to the X-ray diffraction peak attributed to α-CoMoO$_4$, with respect to the peak intensity of the catalyst observed in the range of 2θ = 47.4° ± 0.2° in the X-ray diffraction pattern obtained by using CuKα rays as an X-ray source, it is preferable that a changing amount (D3) per 1000 hours of the reaction time represented by the following formula (13) and the above formulae (4), (10), and (11) is 1.2 or less.

$$D3 = (U3 - F3)/T \times 1000 \quad (13)$$

An upper limit of the changing amount (D3) of the peak intensity observed in the range of 2θ = 47.4° ± 0.2° per 1000 hours of the reaction time is preferably 1.0, 0.50, 0.30, or 0.20 in order, more preferably 0.10, still more preferably 0.050, and most preferably 0.030. A lower limit of the changing amount may not be set, but is preferably -9.6, -9.0, -7.0, -5.0, -3.0, -2.0 in order, more preferably -1.0, still more preferably 0.0, and most preferably 0.010. That is, a more preferred range of the changing amount (D3) of the peak intensity is set by the upper and lower limits described above, and is, for example, -2.0 or more and 1.0 or less, or the like, and most preferably 0.010 or more and 0.050 or less.

[Time T (hr) for which Oxidation Reaction is Carried Out]

The oxidation reaction time T (hr) in the present embodiment is determined with a specific time of 300 hours or more and 30000 hours or less, preferably 800 hours or more and 3000 hours or less, more preferably 1000 hours or more and 1500 hours or less, and most preferably 1300 hours.

However, for the characteristics of the catalyst of the present embodiment, Q1 is particularly preferably 16 or less at any time within the above range of 300 hours or more and 30000 hours or less. It should be noted that when substituting into the above calculation formula, it is preferable to perform the calculation with two significant digits.

The catalyst of the present embodiment preferably has a peak at $2\theta = 27.4° \pm 0.2°$ in addition to the above peak in the X-ray diffraction pattern obtained by using CuKa rays as an X-ray source. When the peak intensity is within a specific range, the catalyst is more preferable. When the peak intensity is defined as S3 shown below, the lower limit of S3 is 11.0, 11.5, or 12.0 in preferred order, and most preferably 12.2, and the upper limit of S3 is 14.0, 13.0, or 12.5 in preferred order, and most preferably 12.4. That is, a preferred range of S3 is 11.0 or more and 14.0 or less, and most preferred range of S3 is 12.2 or more and 12.4 or less.

$$S3 = (\text{peak intensity at } 2\theta = 27.4° \pm 0.2°) / (\text{peak intensity at } 2\theta = 26.5° \pm 0.2°) \times 100$$

In the present embodiment, the effect of the catalyst can be clarified by evaluating and comparing the catalyst before the oxidation reaction and the catalyst after the oxidation reaction under the same evaluation conditions. The evaluation conditions may be any conditions, but the evaluation is preferably performed under a condition in which a propylene space velocity is as large as 300 hr$^{-1}$ or more because it is easy to find a difference between before and after the oxidation reaction.

When the evaluation is performed under the condition of the propylene space velocity of 300 hr$^{-1}$ or more, it is preferable to use a reactor having a small scale because the evaluation can be easily performed. In changing the reactor, when the catalyst is extracted from the reaction tube in order to obtain the catalyst after the oxidation reaction, the reaction tube may be equally divided into three or more sections in the longitudinal direction, and the catalyst may be sampled in equal amounts from the respective positions and mixed, and then the evaluation may be carried out.

[Composition of Catalytically Active Component]

A catalytically active component contained in the catalyst of the present embodiment preferably has a composition represented by the following formula (A).

$$Mo_{a1}Bi_{b1}Ni_{c1}Co_{d1}Fe_{e1}X_{f1}Y_{g1}Z_{h1}O_{i1}... \quad\quad\quad (A)$$

(in the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silicon, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, h1, and i1 represent the number of atoms of molybdenum, bismuth, nickel, cobalt, iron, X, Y, Z, and oxygen, respectively; when a1 = 12, $0 < b1 \leq 7$, $0 \leq c1 \leq 10$, $0 < d1 \leq 10$, $0 < c1 + d1 \leq 20$, $0 \leq e1 \leq 5$, $0 \leq g1 \leq 2$, $0 \leq f1 \leq 3$, $0 \leq h1 \leq 5$, and i1 is a value determined by an oxidation state of each element).

In the formula (1), the preferred ranges of b1 to i1 are as follows.

The lower limit of b1 is 0.2, 0.5, 0.7, or 0.8 in preferred order, and most preferably 0.9, and the upper limit of b1 is 5, 3, 2, 1.6, 1.4, or 1.2 in preferred order, and most preferably 1.1. That is, the most preferred range of b1 is $0.9 \leq b1 \leq 1.1$.

The lower limit of c1 is 1, 2, 2.5, 2.8, or 3.0 in preferred order, and most preferably 3.1, and the upper limit of c1 is 5, 4, 3.8, 3.6, or 3.4 in preferred order, and most preferably 3.2. That is, the most preferred range of c1 is $3.1 \leq c1 \leq 3.2$.

The lower limit of d1 is 3, 4, 5, 5.3, 5.5, or 5.7 in preferred order, and most preferably 5.8, and the upper limit of d1 is 8, 7, 6.5, 6.3, or 6.1 in preferred order, and most preferably 6.0. That is, the most preferred range of d1 is $5.8 \leq d1 \leq 6.0$.

The lower limit of e1 is 0.5, 1, 1.2, or 1.4 in preferred order, and most preferably 1.5, and the upper limit of e1 is 4, 3, 2.5, 2, or 1.8 in preferred order, and most preferably 1.7. That is, the most preferred range of e1 is $1.5 \leq e1 \leq 1.7$.

The upper limit of f1 is 8, 7, 6, or 5 in preferred order. That is, the most preferred range of f1 is $0 \leq f1 \leq 5$.

The lower limit of gl is 0, 0.02, 0.04, or 0.06 in preferred order, and most preferably 0.07, and the upper limit of gl is 1.5, 1, 0.5, 0.2, or 0.15 in preferred order, and most preferably 0.10. That is, the most preferred range of g1 is $0.07 \leq g1 \leq 0.10$.

The upper limit of h1 is 8, 7, 6, or 5 in preferred order. That is, the most preferred range of h1 is $0 \leq h1 \leq 5$.

It is preferable that two or less types of Y are contained, and one type is particularly preferred. In addition, it is particularly preferable that f1 and h1 are 0.

[Carrying]

The catalyst in which a preliminary calcined powder subjected to preliminary calcination after the preparation of the catalytically active component is carried on the inert carrier is particularly excellent as the catalyst of the first aspect.

As the material of the inert carrier, known materials such as alumina, silica, titania, zirconia, niobia, silica alumina, silicon carbide, carbides, and mixtures thereof can be used. Further, the particle size, water absorption rate, mechanical strength, crystallinity of each crystal phase, mixing ratio, etc. are not limited, and an appropriate range of these should be selected in consideration of the final catalyst performance, molding properties, production efficiency, etc. The mixing ratio of the carrier and the preliminarily calcined powder is calculated as the active mass ratio according to the following equation based on the charged mass of each raw material.

$$\text{Active mass ratio (mass\%)} = \text{(mass of preliminary calcined powder used for molding)}/\{\text{(mass of preliminary calcined powder used for molding)} + \text{(mass of carrier used for molding)}\} \times 100$$

The upper limit of the active mass ratio is preferably 80 mass%, and more preferably 60 mass%.

The lower limit is preferably 20 mass%, and more preferably 30 mass%. That is, the most preferred range of the active mass ratio is 30 mass% or more and 60 mass% or less.

As the inert carrier, silica and/or alumina is preferable, and a mixture of silica and alumina is particularly preferable.

For the carrying, it is preferred to use a binder. Specific examples of the binder which can be used include water, ethanol, methanol, propanol, a polyhydric alcohol, polyvinyl alcohol as a polymer-based binder, and a silica sol aqueous solution as an inorganic binder; ethanol, methanol, propanol, and a polyhydric alcohol are preferred; a diol such as ethylene glycol and a triol such as glycerin is preferred; and an aqueous solution having a concentration of glycerin of 5 mass% or more is preferred. Using an appropriate amount of a glycerin aqueous solution allows for obtaining a high-performance catalyst having good molding properties and high mechanical strength. The amount of these binders used is usually 2 to 60 parts by mass with respect to 100 parts by mass of the preliminarily calcined powder. The amount of glycerin aqueous solution is preferably 10 to 30 parts by mass. The binder and the preliminarily calcined powder may be alternately or simultaneously supplied to a molding machine on the occasion of carrying.

For the means for controlling the values of Q1, Q2, Q3, D1, D2, D3 and/or S3, the control can be performed by changing each condition in each production process described later, and examples thereof include (I) a method of changing a catalyst composition, (II) a method of changing a calcination condition, (III) a method of changing a temperature decrease condition after calcination, (IV) a method of controlling a catalyst and a precursor thereof such that the catalyst and the precursor are not subjected to mechanical strength in all the steps of the production of the catalyst, (V) a method of using a raw material having high purity, other methods (VI) to (XI), and a method of combining (I) to (XI). The details of the other methods (VI) to (XI) will be described later.

The method (I) is a method of adjusting d1/(b1 + c1 + e1) in the composition formula (A) to a specific range, and the upper limit of the range is 1.25, preferably 1.20, and more preferably 1.10, and the lower limit of the range is 0.10, 0.30, 0.50, 0.70, 0.80, 0.90, and 1.00 in preferred order. That is, the most preferred range is 1.00 or more and 1.10 or less.

The upper limit of e1/b1 is 1.90, and preferably 1.80; the lower limit of e1/b1 is 0.10, 0.50, 1.00, 1.40, or 1.50 in preferred order; the upper limit of d1/b1 is 9.0, 8.0, 7.0, or 6.0 in preferred order; the lower limit of d1/b1 is 2.0, 3.0, 4.0, 5.0, or 5.5 in preferred order; the upper limit of c1/e1 is 4.0, 3.0, or 2.5 in preferred order; the lower limit of c1/e1 is 1.5, 1.7, or 1.9 in preferred order; the upper limit of c1/d1 is 2.0, 1.0, or 0.8 in preferred order; the lower limit of c1/d1 is 0.4 or 0.5 in preferred order; the upper limit of g1/d1 is 0.20, 0.19, 0.18, 0.17, 0.16, 0.15, 0.14, or 0.10 in preferred order; the lower limit of g1/d1 is 0.01, 0.02, 0.03, 0.04, or 0.05 in preferred order; the upper limit of g1/c1 is 0.041, 0.039, 0.037, 0.035, 0.033, 0.031, 0.029, 0.025, or 0.023 in preferred order; and the lower limit of g1/c1 is 0.017, 0.019 or 0.021 in preferred order. Furthermore, in the composition formula (A), the lower limit of c1 + d1 + e1 is 7.0, 7.5, 8.0, 8.5, 9.0, or 9.5 in preferred order, the upper limit of c1 + d1 + e1 is 13.0, 12.5, 12.0, 11.5, 11.0, or 10.5 in preferred order, the lower limit of b1 + c1 + d1 + e1 is 8.0, 8.5, 9.0, 9.5, 10.0, 10.5, or 11.0 in preferred order, and the upper limit of b1 + c1 + d1 + e1 is 14.0, 13.5, 13.0, 12.5, 12.0, or 11.5 in preferred order.

Regarding the method (II), a temperature in preliminary calcination and main calcination as described later and in both of them is 200°C or more and 600°C or less, preferably 300°C or more and 550°C or less, and more preferably 460°C or more and 550°C or less, and a time in preliminary calcination and main calcination as described later and in both of them is 0.5 hours or more, preferably 1 hour or more and 40 hours or less, more preferably 2 hours or more and 15 hours or less, and most preferably 2 hours or more and 9 hours or less. An atmosphere in preliminary calcination and main calcination as described later and in both of them has 0 vol% or more and 40 vol% or less of the oxygen concentration, preferably 5 vol% or more and 30 vol% or less, more preferably 10% to 25%, and most preferably the atmosphere is an air atmosphere.

Regarding the method (III), in the preliminary calcination and the main calcination as described later and in both of them, a rate of decrease (rate of temperature decrease) of the temperature of a catalyst surface from the maximum temperature (preliminary calcination temperature or main calcination temperature) reached in the calcination step to the room temperature is 1°C/min or more and 200°C/min or less, preferably 5°C/min or more and 150°C/min or less, more preferably 10°C/min or more and 120°C/min or less, and most preferably 50°C/min or more and 100°C/min or less. A temperature decrease method industrially taken in general for achieving the range of the rate of temperature decrease described above, for example, a method of exposing a calcined catalyst taken out from a calcination furnace to an inert atmosphere or a mist of an inert solvent, and a method of rapidly moving a calcined catalyst into a room sufficiently cooled in advance are all included in the present embodiment.

The method (IV) is a method of controlling a catalyst precursor to be described later and/or granules formed in each step such that the catalyst precursor and/or granules are not subjected to a mechanical impact, a shear stress, and the like, and the preferred range of the mechanical impact, shear stress, and the like is controlled to 100 kgf or less, preferably 50 kgf or less, more preferably 20 kgf or less, still more preferably 10 kgf or less, and most preferably 5 kgf or less.

The method (V) is not limited as long as the method (V) is a method using a high purity raw material at the level of reagent, and for example, the content of sulfur and a compound thereof, lithium, halogen and a compound thereof, and lead in the raw material is 10000 ppm by mass or less, preferably 1000 ppm by mass or less, more preferably 100 ppm by mass, and most preferably 10 ppm by mass or less.

The method (VI), as described later, is, for example, a method in which a catalyst precursor is once obtained as granules and the granules are molded. Obtaining the catalyst precursor in the form of granules allows for producing the catalyst such that each component of the catalyst can be more uniform.

The method (VII) is a method of controlling a time during which a cobalt raw material and a nickel raw material are mixed, reacted, slurried, and retained in a mixing pot to be as short as possible in the step of preparing the catalyst to be described later, and more specifically, is a method of shortening the retention time in a state in which a metal salt raw material excluding molybdenum and alkali metal is not in the mixing pot and the cobalt raw material and the nickel raw material are present in the mixing pot, or a method of shortening the retention time in a state in which the cobalt raw material and the nickel raw material are present in the mixing pot when a pH in the mixing pot falls within a specific range. The retention time is preferably 24 hours, more preferably 1 hour, still more preferably 30 minutes, and most preferably 10 minutes. The range of pH is 1 or more and 14 or less, preferably 2 or more and 10 or less, more preferably 2 or more and 8 or less, and most preferably 3 or more and 7 or less. The same applies to an iron raw material and a bismuth raw material, a molybdenum raw material, and a bismuth raw material.

The method (VIII) is a method in which, in the step of preparing a catalyst to be described later, the raw materials are charged in two or more times in a divided manner instead of charging a necessary amount of each raw material at a time. After the divided raw material is charged once, a certain interval is preferably set until the raw material is charged next. The time of the interval is preferably 5 seconds or more and 1 hour or less, more preferably 30 seconds or more and 45 minutes or less, still more preferably 1 minute or less and 30 minutes or less, and most preferably 3 minutes or more and 15 minutes or less. The number of divisions of one raw material is preferably 2 or more, more preferably 3 or more, still more preferably 4 or more, and most preferably 5 or more. Some raw materials can be divided in a series of preparation steps, each raw material may be divided individually, the raw materials may be mixed as described below and then divided collectively, or the raw materials divided individually may be alternately charged.

Regarding the method (IX), when the aqueous solutions of the respective raw materials are mixed and stirred to prepare a suspended slurry in the step of preparing a catalyst to be described later, an adding time of the two or more aqueous solutions used for the mixing is preferably 1 second or more and 30 minutes or less, more preferably 10 seconds or more and 20 minutes or less, still more preferably 30 seconds or more and 5 minutes or less, and most preferably 1 minute or more and 5 minutes or less.

Regarding the method (X), in the step of preparing a catalyst to be described later, a transfer time from preparing the suspended slurry in a final state to moving to the drying step as a next step is preferably 10 seconds or more and 1 hour or less, more preferably 30 seconds or more and 10 minutes or less, and most preferably 1 minute or more and 5 minutes or less.

The method (XI) is a method for adding an organic substance before or after each raw material is added, in the step of preparing a catalyst to be described later, and the lower limit of the amount of the organic substance to be added to the molybdenum raw material is preferably 0.001 mol% or more, more preferably 0.01 mol% or more, still more preferably 0.1 mol% or more, and most preferably 1 mol% or more, and the upper limit of the amount of the organic substance to be added to the molybdenum raw material is preferably 100 mol% or less, more preferably 90 mol% or less, still more preferably 80 mol% or less, and most preferably 60 mol% or less. Carboxylic acids and alcohols are preferable as the organic substance to be added. Examples of the organic substance include acetic acid, propionic acid, lactic acid, citric acid, stearic acid, oleic acid, ethylenediamine tetraacetic acid, methanol, ethanol, propanol, ethylene glycol, and glycerin.

As described above, the nature of the present invention is that the changing rate of the peak intensity of the crystal phase of $\alpha$-CoMoO$_4$ per 1000 hours of the reaction time of the oxidation reaction is equal to or less than a certain value. In addition to consider the catalyst composition as described above, devising the production conditions of the unsaturated aldehyde compound, the unsaturated carboxylic acid compound, or the conjugated diene compound is also useful for maintaining the reaction process stably. That is, the production conditions may be controlled so that the changing rate of the peak intensity of the crystal phase of $\alpha$-CoMoO$_4$ per 1000 hours of the reaction time of the oxidation reaction is a certain value or less. Specifically, examples thereof include (XII) a method of controlling a hot spot temperature of a catalyst layer, (XIII) a method of controlling an oxygen concentration at the outlet of a reaction tube, (XIV) a method of controlling a steam concentration at the inlet of a reaction tube, (XV) a method of controlling a rate of temperature decrease when any process for temperature decrease is performed during the reaction, (XVI) a method of preventing a mechanical impact on the catalyst, and a method of combining the methods (XII) to (XVI).

The method (XII) is a method of controlling the hot spot temperature of the catalyst layer at 427°C or lower for producing an unsaturated aldehyde compound, an unsaturated carboxylic acid compound, and/or a conjugated diene, and the upper limit of the hot spot temperature is preferably 420°C or lower, 410°C or lower, 400°C or lower, 390°C or lower, or 380°C or lower. That is, the hot spot temperature is most preferably 380°C or lower. The time for controlling the hot spot temperature is 500 hours or less, preferably 300 hours or less, more preferably 200 hours or less, still more preferably 100 hours or less, and most preferably 50 hours or less.

The method (XIII) is a method of controlling the oxygen concentration at the outlet of the reaction tube for producing an unsaturated aldehyde compound, an unsaturated carboxylic acid compound, and/or a conjugated diene to 4.0 vol% or more. The lower limit of the oxygen concentration is preferably 4.3 vol% or more, more preferably 4.5 vol% or more, and most preferably 4.7 vol% or more.

The method (XIV) is a method of controlling the steam concentration at the inlet of the reaction tube for producing an unsaturated aldehyde compound, an unsaturated carboxylic acid compound, and/or a conjugated diene to 30 vol% or less. The upper limit of the steam concentration is 25 vol% or less, 20 vol% or less, 15 vol% or less, 10 vol% or less, and 9 vol% or less in preferred order. That is, the steam concentration is most preferably 9 vol% or less.

Regarding the method (XV), a rate of decrease (rate of temperature decrease) of the temperature of the catalyst itself from a salt bath temperature to 100°C or less is 1°C/min or more and 200°C/min or less, preferably 5°C/min or more and 150°C/min or less, more preferably 10°C/min or more and 120°C/min or less, and most preferably 50°C/min or more and 100°C/min or less. In order to achieve the above-described range of the rate of temperature decrease, all methods industrially typically taken for the temperature decrease fall within the present embodiment.

The method (XVI) is a method of controlling the catalyst such that the catalyst is not subjected to a mechanical impact, a shear stress, and the like in any step from the filling of the catalyst to the reaction, and the preferred range of the mechanical impact, the shear stress, and the like is controlled to 100 kgf or less, preferably 50 kgf or less, more preferably 20 kgf or less, still more preferably 10 kgf or less, and most preferably 5 kgf or less.

[Method for Producing Catalyst]

A starting raw material for each element constituting the catalyst of the present embodiment and the preliminary calcined powder thereof is not limited. For example, as a raw material of a molybdenum component, molybdenum oxides such as molybdenum trioxide, molybdic acid or salts thereof such as molybdate, ammonium paramolybdate and ammonium metamolybdate, and heteropolyacids containing molybdenum or salts thereof such as phosphomolybdic acid and silicate molybdic acid, can be used.

As a raw material of a bismuth component, bismuth salts such as bismuth nitrate, bismuth carbonate, bismuth sulfate, and bismuth acetate, bismuth trioxide, metal bismuth and the like can be used. These raw materials can be used as solids or as an aqueous solution, a nitric acid solution, or a slurry of bismuth compounds generated from those aqueous solutions, and the nitrate, a solution thereof, or a slurry obtained from the solution is preferably used.

As a starting raw material for other constituent elements, ammonium salt, nitrate, nitrite, carbonate, subcarbonate, acetate, chloride, inorganic acid, inorganic acid salt, heteropolyacid, heteropolyacid salt, sulfate, hydroxide, organic acid salt, and oxide of metallic elements commonly used in this type of catalyst may be used, or a mixture thereof may be used in combination. Ammonium salts and nitrates are preferably used.

A compound containing these active components may be used alone or in combination of two or more. A slurry liquid can be obtained by uniformly mixing each compound containing an active component and water. The amount of water to be used in the slurry liquid is not limited as long as the total amount of the compound to be used can be completely dissolved or uniformly mixed. The amount of water to be used may be appropriately determined in consideration of the drying method and the drying conditions. Usually, the amount of water to be used is 100 parts by mass or more and 2000 parts by mass or less with respect to 100 parts by mass of the total mass of the compound for preparing a slurry. The amount of water may be large, but too large amount of water causes many disadvantages such as an increase in the energy cost of the drying step and a possible failure to completely dry.

The slurry liquid of the source compound of the above each component element is preferably prepared by (a) a method of mixing each of the above source compounds at once, (b) a method of mixing the above source compounds at once and then performing aging, (c) a method of mixing the above source compounds stepwise, (d) a method of repeating mixing step and aging step stepwise, and (e) a method combining (a) to (d). Here, the above aging means "an operation in which industrial raw materials or semi-finished products are processed under specific conditions such as a certain period of time and a certain temperature to conduct acquisition or improvement of the required physical properties and chemical properties, or proceeding of a predetermined reaction". In the present embodiment, the above certain period of time means a range of 5 minutes or longer and 24 hours or shorter, and the above certain temperature means a range from room temperature to a point equal to or lower than a boiling point of an aqueous solution or an aqueous dispersion liquid. Among these, in terms of the activity and yield of the finally obtained catalyst, preferred is the (c) method of mixing the above source compounds stepwise, more preferred is a method in which each raw material to be mixed with a mother liquid stepwise is completely dissolved to be a solution, and most preferred is a method of mixing various mixed solutions of alkali metal solution and nitrate with a mother liquid in which the raw material of the molybdenum is a mixed solution or slurry. However, it is not always necessary to mix all the elements constituting the catalyst in this step, and some elements or some amounts thereof may be added in the subsequent steps.

In the present embodiment, the shape of the stirring blade of the stirrer used in mixing the essential active components is not limited. Any stirring blade such as a propeller blade, a turbine blade, a paddle blade, an inclined paddle blade, a screw blade, an anchor blade, a ribbon blade, a large lattice blade can be used in one stage or in two or more stages of which blades are the same or different types in the vertical direction. In addition, a baffle (obstruction plate) may be installed in the reaction tank if necessary.

Then, the slurry liquid thus-obtained is dried. The drying method is not limited so long as the slurry liquid can be completely dried by the method, but examples thereof include drum drying, freeze drying, spray drying and evaporation drying. Among these, spray drying, which allows the slurry liquid to be dried into a powder or granule within a short period of time, is particularly preferred in the present embodiment. The temperature of the drying with spray drying varies depending on the concentration of the slurry liquid, the liquid sending speed, or the like. Typically, the temperature at the outlet of a drying machine is 70°C or higher and 150°C or lower.

Subjecting the catalyst precursor obtained as described above to preliminary calcination, molding, and then main calcination allows for controlling and holding the obtained shape, and obtaining a catalyst having particularly excellent mechanical strength for industrial use, and the catalyst can exhibit stable catalyst performance.

As for the molding, either a carrying shaping in which the preliminarily calcined powder is carried on a carrier such as silica or a non-carrying shaping in which no carrier is used can be adopted. Specific examples of the molding method include tablet molding, press molding, extrusion molding and granulation molding. As the shape of the molded product, for example, a columnar shape, a ring shape, a spherical shape or the like can be appropriately selected in consideration of operating conditions. Preferred is a carried catalyst in which a catalytically active component is carried on a spherical carrier, particularly an inert carrier such as silica or alumina and in which the average particle size is 3.0 mm or more and 10.0 mm or less, and preferably 3.0 mm or more and 8.0 mm or less. As for the carrying method, a tumbling granulation method, a method using a centrifugal flow coating apparatus, a wash coating method, and the like are widely known. The method is not limited as long as the preliminarily calcined powder can be uniformly carried on the carrier, but the tumbling granulation method is preferred in consideration of the production efficiency of the catalyst and the like. Specifically, the tumbling granulation method is a method in which using a device that has a flat or uneven disk at the bottom of a fixed cylindrical container, a carrier charged into the container is vigorously agitated by means of a repeat of rotation motion and revolution motion of the carrier itself by rotating the disk at a high speed, and then the preliminarily calcined powder is added into the container to carry the powder component on the carrier.

On the occasion of carrying, it is preferred to use a binder. Specific examples of the binder which can be used include water, ethanol, methanol, propanol, a polyhydric alcohol, polyvinyl alcohol as a polymer-based binder and a silica sol aqueous solution as an inorganic binder; ethanol, methanol, propanol, and a polyhydric alcohol are preferred; a diol such as ethylene glycol and a triol such as glycerin is more preferred; and an aqueous solution of glycerin having a concentration of 5 mass% or more is still more preferred. Using an appropriate amount of the glycerin aqueous solution allows for obtaining a high-performance catalyst having good molding properties and high mechanical strength. The amount of these binders to be used is usually 2 to 60 parts by mass with respect to 100 parts by mass of the preliminarily calcined powder, and the amount of the glycerin aqueous solution is preferably 15 to 50 parts by mass. The binder and the preliminarily calcined powder may be alternately supplied to a molding machine or simultaneously supplied to the molding machine on the occasion of the carrying. Further, on the occasion of molding, a small amount of known additives such as graphite and talc may be added. None of a molding aid, a pore-forming agent and a carrier added in the molding is considered as the constituent element of the active component in the present embodiment, regardless of whether the molding aid, the pore-forming agent and the carrier have the activity in the sense of converting the raw material into some other product.

The preliminary calcination method, the preliminary calcination conditions, the main calcination method, and the main calcination conditions are not limited, but known treatment methods and conditions can be applied. The preliminary calcination or the main calcination is usually carried out at 200°C or higher and 600°C or lower, and preferably 300°C or higher and 550°C or lower, for 0.5 hours or longer, and preferably 1 hour or longer and 40 hours or shorter under the conditions that an oxygen-containing gas such as air or an inert gas flow. Here, the inert gas refers to a gas that does not reduce the reaction activity of the catalyst, and specific examples thereof include nitrogen, carbon dioxide, helium and argon. The optimum conditions for the main calcination vary depending on the reaction conditions when an unsaturated aldehyde and/or an unsaturated carboxylic acid are produced using a catalyst, and changing the process parameters of the main calcination step, that is, the oxygen content in the atmosphere, the maximum temperature reached and the calcination time falls within the scope of the present embodiment, because the changing is well-known for the skilled person. The main calcination step shall be carried out after the above preliminary calcination step, and the maximum temperature reached (main calcination temperature) in the main calcination step shall be higher than the maximum temperature reached (preliminary calcination temperature) in the above preliminary calcination step. The technique of the calcination includes but not limited to a fluidized bed, rotary kiln, muffle furnace, and tunnel firing furnace, and should be selected within an appropriate range in consideration of the final catalyst performance, mechanical strength, molding properties, production efficiency and the like.

The catalyst of the present embodiment is preferably used as a catalyst for producing an unsaturated aldehyde compound, an unsaturated carboxylic acid compound, or a conjugated diene compound, is more preferably used as a catalyst for producing an unsaturated aldehyde compound, and is particularly preferably used as a catalyst for producing acrolein from propylene. In a process of an exothermic reaction such as production of an unsaturated aldehyde compound, an unsaturated carboxylic acid compound, or a conjugated diene compound, it is known to those skilled in the art that different catalyst types are filled in multiple layers so that the activity is increased from an inlet side of a reaction tube toward an outlet side of the reaction tube, for the purpose of preventing deterioration of the catalyst itself due to heat generated by the reaction in an actual plant. The catalyst of the present embodiment can be used on either an inlet side of the reaction tube, an outlet side of the reaction tube, or the middle catalyst layer. For example, the catalyst of the present embodiment is most preferably used on the most outlet side of the reaction tube, that is, the catalyst of the present embodiment is used as the most active catalyst among all catalyst layers in the reaction tube. For the multilayer filling, two-layer or three-layer filling is particularly preferred.

[Catalyst in Second Stage]

When the catalyst of the present embodiment is used as a catalyst in a first stage, that is, a catalyst for producing an unsaturated aldehyde compound, an unsaturated carboxylic acid compound can be obtained by performing a second-stage oxidation reaction.

In this case, the catalyst of the present embodiment can also be used as a catalyst in a second stage, but a catalyst containing a catalytically active component represented by the following formula (B) is preferred.

$$Mo_{12}V_{a2}W_{b2}Cu_{c2}Sb_{d2}X2_{e2}Y2_{f2}Z2g_2O_{b2}... \qquad (B)$$

(In the formula, Mo, V, W, Cu, Sb and O represent molybdenum, vanadium, tungsten, copper, antimony and oxygen, respectively; X2 represents at least one element selected from the group consisting of an alkali metal and thallium; Y2 represents at least one element selected from the group consisting of magnesium, calcium, strontium, barium and zinc; and Z represents at least one element selected from the group consisting of niobium, cerium, tin, chromium, manganese, iron, cobalt, samarium, germanium, titanium and arsenic. a2, b2, c2, d2, e2, f2, g2 and h2 represent the atomic proportion of each element, and with respect to molybdenum atom 12, a2 satisfies $0 < a2 \leq 10$, b2 satisfies $0 \leq b2 \leq 10$, c2 satisfies $0 < c2 \leq 6$, d2 satisfies $0 < d2 \leq 10$, e2 satisfies $0 \leq e2 \leq 0.5$, f2 satisfies $0 \leq f2 \leq 1$, and g2 satisfies $0 \leq g2 < 6$. Further, h2 is the number of oxygen atoms required to satisfy the atomic value of each component.)

In the production of a catalyst containing the catalytically active component represented by the above formula (B), a method widely known as a method for preparing this kind of a catalyst, for example, an oxide catalyst or a catalyst having a heteropolyacid or a salt structure thereof, can be adopted. The raw materials that can be used in producing the catalyst are not limited, and various materials can be used. For example, molybdenum oxides such as molybdenum trioxide, molybdic acid or salts thereof such as molybdic acid and an ammonium molybdate, molybdenum-containing heteropolyacids or salts thereof such as phosphomolybdic acid and silicomolybdic acid, and the like can be used. The raw material of an antimony component is not limited, but antimony trioxide or antimony acetate is preferred. As raw materials for other elements such as vanadium, tungsten, copper and the like, nitrate, sulfate, carbonate, phosphate, organic acid salt, halide, hydroxide, oxide or the metal of these elements can be used.

A compound containing these active components may be used alone or in combination of two or more.

Next, the slurry liquid obtained above is dried to obtain a solid of catalytically active component. The drying method is not limited so long as the slurry liquid can be completely dried by the method. However, examples thereof include drum drying, freeze drying, spray drying and evaporation drying. Spray drying is preferred because the slurry liquid can be dried into a powder or granule in a short period of time. The temperature of the drying with spray drying varies depending on the concentration of the slurry liquid, the liquid sending speed or the like, but the temperature at the outlet of a drying machine is approximately 70°C to 150°C. In this case, the slurry liquid is preferably dried such that the average particle size of a slurry liquid dried product (catalyst precursor) to be obtained is 10 $\mu$m to 700 $\mu$m.

The solid of catalytically active component in the second stage obtained as described above can be used as it is for a coating mixture, and is preferably subjected to calcination because the molding properties may be improved. The calcination method and the calcination conditions are not limited, and known treatment methods and conditions can be applied. The optimum calcination conditions vary depending on the used raw material for the catalyst, catalyst composition, preparation method and the like. The calcination temperature is usually 100°C to 350°C, preferably 150°C to 300°C, and the calcination time is usually 1 to 20 hours. The calcination is usually carried out in an air atmosphere, but may be carried out in an atmosphere of an inert gas such as nitrogen, carbon dioxide, helium or argon. The calcination in an air atmosphere may be carried out after calcination in an inert gas atmosphere, if necessary. The thus-obtained calcined solid is preferably pulverized before the molding. The pulverizing method is not limited, but it is preferable to use a ball mill.

The compound containing the active component in preparing the slurry for the second stage does not necessarily have to contain all the active components, and a part of the components may be used before the following molding step.

The shape of the catalyst in the second stage is not limited. The catalyst is used by being molded into a columnar shape, a tablet, a ring shape, a spherical shape or the like in order to reduce the pressure loss of a reaction gas in the oxidation reaction. Among these, the solid of catalytically active component is particularly preferably carried on an inert carrier to be a carried catalyst because improvement in selectivity and removal of heat of reaction can be expected. A tumbling granulation method described below is preferred for the carrying. This method is a method in which, for example, in a device that has a flat or uneven disk at the bottom of a fixed container, a carrier in the container is vigorously agitated by repeatedly performing rotation motion and revolution motion by rotating the disk at a high speed, and then a mixture for the carrying including the binder, the solid of catalytically active component and optionally a molding aid and/or a strength improver is carried on the carrier. As a method of adding the binder, any methods may be adapted such as 1) premixing a binder with the mixture for the carrying, 2) adding the binder at the same time as the mixture for the carrying is added into the fixed container, 3) adding the binder after adding the mixture for the carrying into the fixed container, 4) adding the binder before adding the mixture for the carrying into the fixed container, 5) dividedly preparing the mixture for the carrying and the binder independently and adding the whole amount of them in the appropriate combination of 2) to 4). Among these, for example, 5) is preferably performed by adjusting the adding rate using an auto feeder or the like such that the mixture for the carrying does not adhere to the wall of the fixed container and the mixture for the carrying does not aggregate with each other and a predetermined amount of the mixture for the carrying is carried on the carrier. Examples of the binder include water, ethanol, polyhydric alcohol, polyvinyl alcohol as a polymer-based binder, celluloses such as a crystalline cellulose, methyl cellulose and ethyl cellulose, and an aqueous silica sol solution as an inorganic binder. Diols such as cellulose and ethylene glycol and triols such as glycerin are preferred, and an aqueous solution having a concentration of glycerin of 5 mass% or more is particularly preferred. The amount of these binders to be used is usually 2 to 60 parts by mass, preferably 10 to 50 parts by mass, per 100 parts by mass of the mixture for the carrying.

Specific examples of the carrier in the above carrying include a spherical carrier having a diameter of 1 mm to 15 mm, and preferably 2.5 mm to 10 mm, such as silicon carbide, alumina, silica alumina, mullite and arandom. The carriers having a porosity of 10% to 70% are usually used. The carrier and the mixture for the carrying at the ratio of the mixture for the carrying/(mixture for the carrying + carrier) =10 mass% to 75 mass% are usually used, and the carrier and the mixture for the carrying at the ratio of the mixture for the carrying/(mixture for the carrying + carrier) =15 mass% to 60 mass% are preferably used. When the ratio of the mixture for the carrying tends to be large, the reaction activity of the carried catalyst is large, but the mechanical strength tends to be small. On the contrary, when the ratio of the mixture for the carrying is small, the mechanical strength tends to be large, but the reaction activity tends to be small. In the above, examples of the molding aid to be used as necessary include silica gel, diatomite, and alumina powder. The amount of the molding aid to be used is usually 1 to 60 parts by mass with respect to 100 parts by mass of the solid of catalytically active component. If necessary, the use of inorganic fibers (for example, ceramic fibers or whiskers) that are inactive to the solid of catalytically active component and the reaction gas as the strength improver is useful for improving the mechanical strength of the catalyst, and glass fibers are preferred. The amount of the fiber to be used is usually 1 to 30 parts by mass with respect to 100 parts by mass of the solid of catalytically active component. None of a molding aid, a pore-forming agent and a carrier added in the molding of the catalyst for the first stage is considered as the constituent element of the active component in the present embodiment, regardless of whether the molding aid, the pore-forming agent and the carrier have the activity in the sense of converting the raw material into some other product.

The carried catalyst obtained as described above can be used as a catalyst for the catalytic gas phase oxidation, and is preferably subjected to calcination because the molding properties may be improved. The calcination method and the calcination conditions are not limited, and known treatment methods and conditions can be applied. The optimum calcination conditions vary depending on the raw material for the catalyst to be used, the catalyst composition, the preparation method, and the like, but the calcination temperature is usually 100°C to 450°C, preferably 270°C to 420°C, and the calcination time is usually 1 to 20 hours. The calcination is usually carried out in an air atmosphere, and may be carried out in an atmosphere of an inert gas such as nitrogen, carbon dioxide, helium or argon. The calcination in an air atmosphere may be carried out after the calcination in an inert gas atmosphere, if necessary.

When the catalyst of the present embodiment is used in a reaction of using propylene, isobutylene, t-butyl alcohol and the like as raw materials to produce the corresponding unsaturated aldehyde, unsaturated carboxylic acid, and particularly, in a reaction of producing acrolein and acrylic acid by catalytic gas phase oxidation of propylene with molecular oxygen or a gas containing molecular oxygen, using the catalyst of the present embodiment allows for improving the catalytic activity and the yield, and is very effective in improving the price competitiveness of the product as compared with the known method. In addition, the effect of improving the process stability of the partial oxidation reaction accompanied by heat generation, such as reduction of the hot spot temperature can be expected. Further, the catalyst of the present embodiment is also effective in reducing by-products that adversely influences the environment and the quality of the final product, such as carbon monoxide (CO), carbon dioxide ($CO_2$), acetaldehyde, acetic acid, and formaldehyde.

The thus-obtained catalyst of the present embodiment can be used, for example, for producing acrolein and/or acrylic acid by catalytic gas phase oxidation of propylene using a molecular oxygen-containing gas. In the production method of the present embodiment, the method for flowing the raw material gas may be an ordinary single-flow method or a recycling method, and can be carried out under widely used conditions, and is not limited. For example, a mixed gas containing 1 vol% to 10 vol% and preferably 4 vol% to 9 vol% of propylene, 3 vol% to 20 vol% and preferably 4 vol% to 18 vol% of molecular oxygen, 0 vol% to 60 vol% and preferably 4 vol% to 50 vol% of water vapor at room temperature as a starting raw material, and 20 vol% to 80 vol% and preferably 30 vol% to 60 vol% of an inert gas such as carbon dioxide and nitrogen is introduced to the catalyst of the present embodiment filled in a reaction tube at 250°C to 450°C under normal pressure to 10 atm and a space velocity of 300 to 5000 $h^{-1}$ to perform a reaction.

In the present invention, unless otherwise specified, the improvement of the catalytic activity means that the conversion rate of the raw material is high when the catalytic reaction is carried out at the same salt bath temperature.

In the present invention, unless otherwise specified, a high yield means that the total yield of the corresponding unsaturated aldehyde and/or unsaturated carboxylic acid is high when the oxidation reaction is performed using propylene, isobutylene, t-butyl alcohol, and the like as raw materials. Unless otherwise specified, the yield refers to a useful yield described later.

In the present invention, unless otherwise specified, the constituent elements of the catalytically active component refer to all the elements to be used in the method for producing a catalyst, but the raw materials and the constituent elements thereof that disappear, sublimate, volatilize, and burn at the maximum temperature or lower in the main calcination step are not included in the constituent elements of the catalytically active component. Further, silicon and the other elements constituting inorganic materials contained in the molding aid and the carrier in the shaping step are not included in the constituent elements of the catalytically active component.

In the present invention, the hot spot temperature refers to the maximum temperature in the temperature distribution in the catalyst-filled bed that is measured in thermocouples installed in the multi-tube reaction tube in the long axis direction, and the salt bath temperature refers to a set temperature of a heat medium used for the purpose of cooling the heat generated in the reaction tube. The number of measuring points in the temperature distribution is not limited, but for example, the catalyst filling length is evenly divided from 10 to 1000.

Further, in the measurement of the hot spot temperature, it is well-known to a person skilled in the art that a temperature sensor sheath is installed in a long axis direction the reaction tube, and the thermocouple is installed therein, for the purpose of stabilizing the measurement by the thermocouples. An outer diameter of the temperature sensor sheath is not limited, but is, for example, preferably 7 mm or less, more preferably 6 mm or less, and even more preferably 3.5 mm or less, and the outer diameter of the thermocouple is also not limited, but is, for example, preferably 6 mm or less, more preferably 4 mm or less, and even more preferably 3 mm or less.

In the present invention, the unsaturated aldehyde and the unsaturated aldehyde compound refers to organic compounds having at least one double bond and at least one aldehyde in the molecule, such as acrolein and methacrolein. In the present invention, the unsaturated carboxylic acid and the unsaturated carboxylic acid compound refers to organic compounds having at least one double bond and at least one carboxy group or an ester group of the carboxyl group in the molecule, and are, for example, acrylic acid, methacrylic acid, and methyl methacrylate. In the present invention, the conjugated diene refers to a diene in which a double bond is separated by one single bond and which is chemically conjugated, and is, for example, 1,3-butadiene.

The catalyst of the present invention also has an advantage that the activity is stable even when (i) the hot spot temperature is reduced and (ii) the salt bath temperature is low.

[Example]

Hereinafter, the present invention will be described in more detail with reference to Examples. In Examples, a conversion rate of a raw material, a useful selectivity, a butadiene selectivity, an active mass ratio, and an oxygen concentration at an outlet were calculated according to the following formulae.

$$\text{Conversion rate (\%) of raw material} = \text{(number of moles of reacted propylene,}$$
$$\text{t-butyl alcohol, isobutylene or butene)/(number of moles of supplied propylene, t-butyl}$$
$$\text{alcohol, isobutylene or butene)} \times 100$$

$$\text{Useful selectivity (\%)} = \text{(total number of moles of produced acrolein and acrylic}$$
$$\text{acid, or total number of moles of produced methacrolein and methacrylic acid)/(number of}$$
$$\text{moles of reacted propylene, t-butyl alcohol or isobutylene)} \times 100$$

$$\text{Butadiene selectivity (\%)} = \text{(total number of moles of produced butadiene)/(number}$$
$$\text{of moles of reacted butene)} \times 100$$

$$\text{Active mass ratio (mass\%)} = \text{(mass of preliminary calcined powder used for}$$
$$\text{molding)/\{(mass of preliminary calcined powder used for molding)} + \text{(mass of carrier used}$$
$$\text{for molding)\}} \times 100$$

$$\text{Oxygen concentration (vol\%) at outlet} = \text{(number of moles of oxygen at outlet of}$$
$$\text{reaction tube)/(number of moles of total gas at outlet of reaction tube containing water vapor)}$$
$$\times 100$$

An X-ray diffraction (XRD) angle ($2\theta$) was measured by using Ultima IV manufactured by Rigaku Corporation under conditions of an X-ray CuKa ray ($\lambda = 0.154$ nm), an output of 40 kV, 30 mA, a measurement range of 10° to 60°, and a measurement rate of 10° per minute. Further, a calcination time described in each of the following examples means a holding time from a time when each calcination temperature reaches, in which a time of temperature increase and temperature decrease is not included. In addition, an aging treatment described later means that a reaction tube having a specified thickness is filled with a catalyst, propylene is caused to flow at a specified flow rate, and an oxidation reaction is performed for a specified period. At this time, the salt bath temperature is any temperature, but the lower limit is 300°C, and the upper limit is a temperature at which the temperature of the catalyst layer in the reaction tube is 450°C or less.

[Example 1]

100 parts by mass of ammonium heptamolybdate was completely dissolved in 380 parts by mass of pure water heated to 60°C (mother liquid 1). Next, 0.37 parts by mass of potassium nitrate was dissolved in 3.3 parts by mass of pure water, and the mixture was added to the mother liquid 1. Next, 31 parts by mass of ferric nitrate, 81 parts by mass of cobalt nitrate, and 44 parts by mass of nickel nitrate were dissolved in 83 parts by mass of pure water heated to 60°C, and the mixture was added dropwise to the mother liquid 1. Subsequently, 23 parts by mass of bismuth nitrate was dissolved in an aqueous nitric acid solution prepared by adding 5.8 parts by mass of nitric acid (60 mass%) to 24 parts by mass of pure water heated to 60°C, and the mixture was added dropwise to the mother liquid 1. The mother liquid 1 was dried by spray drying, and the obtained dried powder was preliminary calcined at 440°C for 4 hours. Five mass% of a crystalline cellulose with respect to the preliminarily calcined powder (the atomic proportion calculated from the charged raw materials was Mo:Bi:Fe:Co:Ni:K = 12:1.0:1.6:5.9:3.2:0.080) was added to the preliminarily calcined powder, followed by thoroughly being mixed. The mixture was carried and molded into a spherical shape on an inert carrier by using a 33 mass% glycerin solution as a binder by tumbling granulation so that an active mass ratio was 50 mass%.

The thus-obtained spherical molded product having a particle size of 5.3 mm was subjected to main calcination under the conditions of 510°C and 4 hours to obtain a catalyst 1-1. The X-ray diffraction angle ($2\theta$) of the catalyst 1-1 was measured. S3 was 12.3.

A stainless steel reaction tube having an inner diameter of 25 mm was filled with the catalyst 1-1, and an aging treatment was carried out for 1300 hours under the conditions of a propylene concentration of 8 vol% and a propylene space velocity of 160 hr$^{-1}$ with respect to all the catalysts in the reaction tube. The maximum value of the temperature of the catalyst layer in the reaction tube during the aging treatment was 444°C, and the minimum value of the oxygen concentration of the gas at the outlet of the reaction tube was 4.8 vol%. Thereafter, the mixture was taken out of the reaction tube to obtain a catalyst 1-2. The X-ray diffraction angle ($2\theta$) of the catalyst 1-2 was measured.

[Example 2]

100 parts by mass of ammonium heptamolybdate was completely dissolved in 380 parts by mass of pure water heated to 60°C (mother liquid 1). Next, 0.17 parts by mass of potassium nitrate was dissolved in 1.5 parts by mass of pure water, and the mixture was added to the mother liquid 1. Next, 38 parts by mass of ferric nitrate, 89 parts by mass of cobalt nitrate, and 33 parts by mass of nickel nitrate were dissolved in 85 parts by mass of pure water heated to 60°C, and the mixture was added dropwise to the mother liquid 1. Subsequently, 21 parts by mass of bismuth nitrate was dissolved in an aqueous nitric acid solution prepared by adding 5.4 parts by mass of nitric acid (60 mass%) to 23 parts by mass of pure water heated to 60°C, and the mixture was added dropwise to the mother liquid 1. The mother liquid 1 was dried by spray drying, and the obtained dried powder was preliminary calcined at 440°C for 4 hours. Five mass% of a crystalline cellulose with respect to the preliminarily calcined powder (the atomic proportion calculated from the charged raw materials was Mo:Bi:Fe:Co:Ni:K = 12:0.93:2.0:6.5:2.4:0.040) was added to the preliminarily calcined powder, followed by thoroughly being mixed. The mixture was carried and molded into a spherical shape on an inert carrier by using a 33 mass% glycerin solution as a binder by tumbling granulation so that an active mass ratio was 50 mass%. The thus-obtained spherical molded product having a particle size of 5.3 mm was subjected to main calcination under the conditions of 550°C and 4 hours to obtain a catalyst 2-1. The X-ray diffraction angle ($2\theta$) of the catalyst 2-1 was measured. S3 was 11.3.

A stainless steel reaction tube having an inner diameter of 25 mm was filled with the catalyst 2-1, and an aging treatment was carried out for 26000 hours under the conditions of a propylene concentration of 8 vol% and a propylene space velocity of 95 hr$^{-1}$ with respect to all the catalysts in the reaction tube. The maximum value of the temperature of the catalyst layer in the reaction tube during the aging treatment was 384°C, and the minimum value of the oxygen concentration of the gas at the outlet of the reaction tube was 3.9 vol%. Thereafter, the mixture was taken out of the reaction tube to obtain a catalyst 2-2. The X-ray diffraction angle ($2\theta$) of the catalyst 2-2 was measured.

[Example 3]

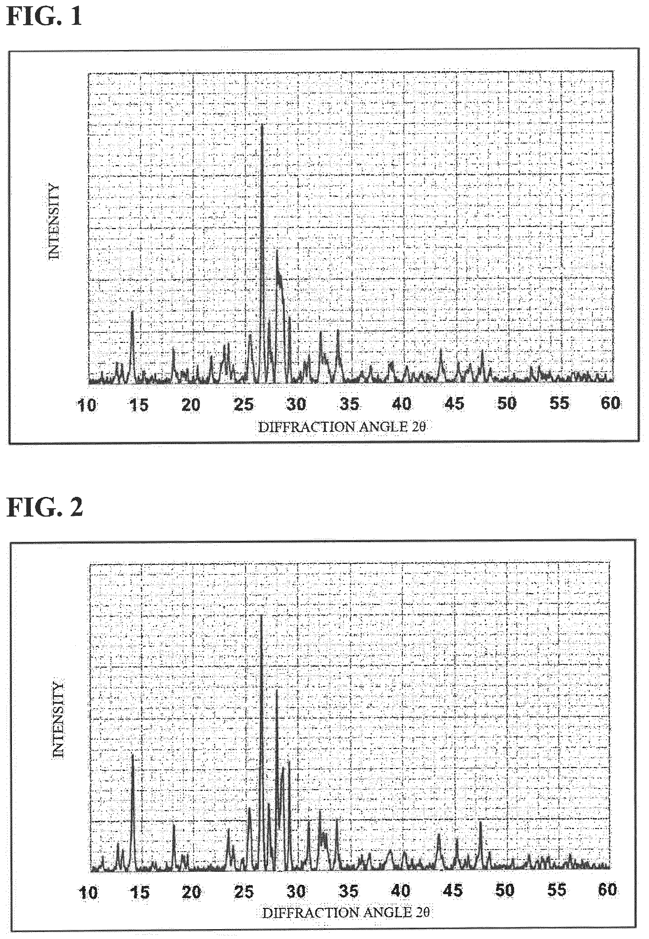

100 parts by mass of ammonium heptamolybdate was completely dissolved in 380 parts by mass of pure water heated to 60°C (mother liquid 1). Next, 0.44 parts by mass of potassium nitrate was dissolved in 4.0 parts by mass of pure water, and the mixture was added to the mother liquid 1. Next, 34 parts by mass of ferric nitrate, 71 parts by mass of cobalt nitrate, and 38 parts by mass of nickel nitrate were dissolved in 76 parts by mass of pure water heated to 60°C, and the mixture was added to the mother liquid 1. Subsequently, 38 parts by mass of bismuth nitrate was dissolved in an aqueous nitric acid solution prepared by adding 9.9 parts by mass of nitric acid (60 mass%) to 41 parts by mass of pure water heated to 60°C, and the mixture was added to the mother liquid 1. The mother liquid 1 was dried by spray drying, and the obtained dried powder was preliminary calcined at 440°C for 4 hours. Five mass% of a crystalline cellulose with respect to the preliminarily calcined powder (the atomic proportion calculated from the charged raw materials was Mo:Bi:Fe:Co:Ni:K = 12:1.7:1.8:5.2:2.8:0.095) was added to the preliminarily calcined powder, followed by thoroughly being mixed. The mixture was carried and molded into a spherical shape on an inert carrier by using a 33 mass% glycerin solution as a binder by tumbling granulation so that an active mass ratio was 50 mass%. The thus-obtained spherical molded product having a particle size of 5.3 mm was subjected to main calcination under the conditions of 530°C and 4 hours to obtain a catalyst 3-1. The X-ray diffraction angle ($2\theta$) of the catalyst 3-1 was measured. FIG. 1 is a diagram illustrating an X-ray diffraction pattern of the catalyst 3-1. S3 was 12.0.

A stainless steel reaction tube having an inner diameter of 27 mm was filled with the catalyst 3-1, and an aging treatment was carried out for 24000 hours under the conditions of a propylene concentration of 8 vol% and a propylene space velocity of 100 hr$^{-1}$ with respect to all the catalysts in the reaction tube. Thereafter, the mixture was taken out of the reaction tube to obtain a catalyst 3-2. The X-ray diffraction angle ($2\theta$) of the catalyst 3-2 was measured. FIG. 2 is a diagram illustrating an X-ray diffraction pattern of the catalyst 3-2.

[Comparative Example 1]

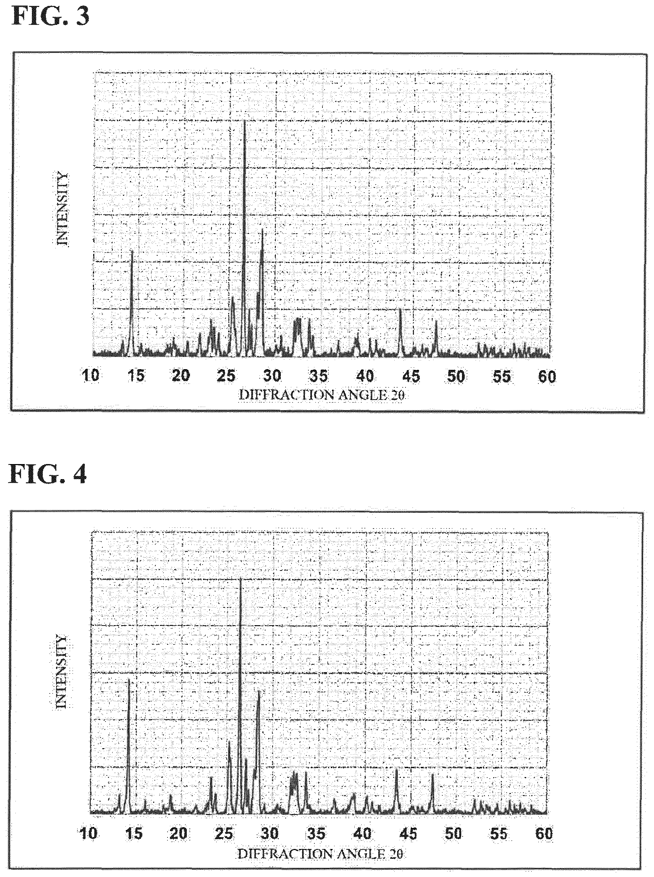

100 parts by mass of ammonium heptamolybdate was completely dissolved in 380 parts by mass of pure water heated to 60°C (mother liquid 1). Next, 0.46 parts by mass of potassium nitrate was dissolved in 4.1 parts by mass of pure water, and the mixture was added to the mother liquid 1. Next, 38 parts by mass of ferric nitrate, 89 parts by mass of cobalt nitrate, and 33 parts by mass of nickel nitrate were dissolved in 85 parts by mass of pure water heated to 60°C, and the mixture was added to the mother liquid 1. Subsequently, 16 parts by mass of bismuth nitrate was dissolved in an aqueous nitric acid solution prepared by adding 4.1 parts by mass of nitric acid (60 mass%) to 17 parts by mass of pure water heated to 60°C, and the mixture was added to the mother liquid 1. The mother liquid 1 was dried by spray drying, and the obtained dried powder was preliminary calcined at 440°C for 4 hours. Five mass% of a crystalline cellulose with respect to the preliminarily calcined powder (the atomic proportion calculated from the charged raw materials was Mo:Bi:Fe:Co:Ni:K = 12:0.7:2.0:6.5:2.4:0.10) was added to the preliminarily calcined powder, followed by thoroughly being mixed. The mixture was carried and molded into a spherical shape on an inert carrier by using a 33 mass% glycerin solution as a binder by tumbling granulation so that an active mass ratio was 50 mass%. The thus-obtained spherical molded product having a particle size of 5.3 mm was subjected to main calcination under the conditions of 540°C and 4 hours to obtain a catalyst 4-1. The X-ray diffraction angle (2θ) of the catalyst 4-1 was measured. FIG. 3 is a diagram illustrating an X-ray diffraction pattern of the catalyst (catalyst 4-1) in Comparative Example 1. S3 was 13.5.

A stainless steel reaction tube having an inner diameter of 25 mm was filled with the catalyst 4-1, and an aging treatment was carried out for 1300 hours under the conditions of a propylene concentration of 8 vol% and a propylene space velocity of 160 hr$^{-1}$ with respect to all the catalysts in the reaction tube. The maximum value of the temperature of the catalyst layer in the reaction tube during the aging treatment was 444°C, and the minimum value of the oxygen concentration of the gas at the outlet of the reaction tube was 4.8 vol%. Thereafter, the mixture was taken out of the reaction tube to obtain a catalyst 4-2. The X-ray diffraction angle (2θ) of the catalyst 4-2 was measured. FIG. 4 is a diagram illustrating an X-ray diffraction pattern of the catalyst 4-2.

Using the catalyst 1-1, the catalyst 2-1, the catalyst 3-1, and the catalyst 4-1, and the catalyst 1-2, the catalyst 2-2, and the catalysts 3-2 and 4-2 taken out from the reaction tube after the aging treatment, the oxidation reaction of propylene was carried out by the following method, and the conversion rate of the raw material and the useful selectivity were determined. A stainless steel reaction tube having an inner diameter of 18.4 mm was filled with each catalyst, and a mixed gas having a gas volume ratio of propylene:oxygen:water vapor = 1:1.7:3.0 was introduced at a propylene space velocity of 400 hr$^{-1}$ with respect to all the catalysts in the reaction tube to carry out an oxidation reaction of propylene. The gas at the outlet of the reaction tube was analyzed between 100 hr and 150 hr from the start of introduction of propylene. The results of the salt bath temperature, the conversion rate of the raw material, the useful selectivity, and the XRD measurement of the catalyst 1-1, the catalyst 2-1, the catalyst 3-1, and the catalyst 1-4 are shown in Table 1, and the results of the salt bath temperature, the conversion rate of the raw material, the useful selectivity, and the XRD measurement of the catalyst 1-2, the catalyst 2-2, the catalyst 3-2, and the catalyst 4-2 are shown in Table 2.

[Table 1]

| | Salt bath temperature | Conversion rate of raw material | Useful selectivity | F1 | F2 | F3 |
|---|---|---|---|---|---|---|
| | (°C) | (%) | (%) | | | |
| Catalyst 1-1 | 400 | 53.9 | 95.5 | 17.10 | 8.75 | 9.70 |
| Catalyst 2-1 | 420 | 60.9 | 94.1 | 25.88 | 11.67 | 10.27 |
| Catalyst 3-1 | 400 | 51.8 | 96.1 | 18.70 | 9.03 | 12.40 |
| Catalyst 4-1 | 400 | 49.7 | 96.6 | 25.22 | 16.32 | 14.98 |

[Table 2]

| | Salt bath temperature | Conversion rate of raw material | Useful selectivity | U1 | U2 | U3 |
|---|---|---|---|---|---|---|
| | (°C) | (%) | (%) | | | |
| Catalyst 1-2 | 400 | 53.8 | 95.4 | 15.34 | 7.30 | 9.73 |
| Catalyst 2-2 | 440 | 59.1 | 91.6 | 29.90 | 16.67 | 14.55 |
| Catalyst 3-2 | 440 | 52.8 | 90.4 | 24.73 | 14.67 | 18.76 |
| Catalyst 4-2 | 400 | 49.3 | 95.1 | 30.67 | 17.36 | 16.70 |

Table 3 shows Q1, Q2, Q3, D1, D2, D3, and the amount of decrease in the useful selectivity per 1000 hours of the reaction time in consideration of the reaction time T (hr) during which the oxidation reaction was carried out.

[Table 3]

| | Catalyst before aging treatment | Catalyst after aging treatment | Amount of decrease in useful selectivity | Q1 | Q2 | Q3 | D1 | D2 | D3 |
| | | | (%) | | | | | | |
|---|---|---|---|---|---|---|---|---|---|
| Example 1 | Catalyst 1-1 | Catalyst 1-2 | -0.077 | -7.92 | -12.75 | 0.24 | -1.35 | -1.12 | 0.02 |
| Example 2 | Catalyst 2-1 | Catalyst 2-2 | -0.096 | 0.60 | 1.65 | 1.60 | 0.15 | 0.19 | 0.16 |
| Example 3 | Catalyst 3-1 | Catalyst 3-2 | -0.24 | 1.34 | 2.60 | 2.14 | 0.25 | 0.24 | 0.27 |
| Comparative Example 1 | Catalyst 4-1 | Catalyst 4-2 | -1.15 | 16.62 | 4.90 | 8.83 | 4.19 | 0.80 | 1.32 |

Although the present invention has been described in detail with reference to specific examples, it is apparent to those skilled in the art that it is possible to add various alterations and modifications without departing from the spirit and the scope of the present invention.

The present application is based on Japanese Patent Application (No. 2020-002508) filed on January 10, 2020, the entire contents of which are incorporated herein by reference. In addition, all references cited here are entirely incorporated.

INDUSTRIAL APPLICABILITY

Using the catalyst of the present invention allows for achieving a high selectivity, for performing a partial oxidation reaction to produce an unsaturated aldehyde compound, an unsaturated carboxylic acid compound, or a conjugated diene compound. As a result, achieving a high yield is expected.

**Claims**

1. A catalyst, comprising, as an essential component,

molybdenum;
bismuth;
and cobalt, wherein
with respect to a peak intensity at $2\theta = 25.3° \pm 0.2°$ in an X-ray diffraction pattern obtained by using CuKa rays as an X-ray source, a changing rate (Q1) per 1000 hours of reaction time represented by the following formulae (1) to (4) is 16 or less.

$$Q1 = \{(U1/F1 - 1) \times 100\}/T \times 1000 \quad (1)$$

$$F1 = \text{(peak intensity of catalyst before oxidation reaction at } 2\theta = 25.3° \pm 0.2°)/\text{(peak intensity of catalyst before oxidation reaction at } 2\theta = 26.5° \pm 0.2°) \times 100 \quad (2)$$

$$U1 = \text{(peak intensity of catalyst after oxidation reaction at } 2\theta = 25.3° \pm 0.2°)/\text{(peak intensity of catalyst after oxidation reaction at } 2\theta = 26.5° \pm 0.2°) \times 100 \quad (3)$$

$$T = \text{time (hr) during which oxidation reaction is carried out} \quad (4)$$

2. The catalyst according to claim 1, wherein with respect to a peak intensity at $2\theta = 25.3° \pm 0.2°$ in an X-ray diffraction pattern obtained by using CuKa rays as an X-ray source, a changing amount (D1) per 1000 hours of reaction time represented by the following formula (5) and the formulae (2) to (4) is 4.1 or less.

$$D1 = (U1 - F1)/T \times 1000 \quad (5)$$

3. The catalyst according to claim 1 or 2, wherein a composition of a catalytically active component is represented by the following formula (A):

$$Mo_{a1}Bi_{b1}Ni_{c1}Co_{d1}Fe_{e1}X_{f1}Y_{g1}Z_{h1}O_{i1} \quad (A)$$

(in the formula, Mo, Bi, Ni, Co and Fe represent molybdenum, bismuth, nickel, cobalt and iron, respectively; X is at least one element selected from tungsten, antimony, tin, zinc, chromium, manganese, magnesium, silica, aluminum, cerium and titanium; Y is at least one element selected from sodium, potassium, cesium, rubidium, and thallium; Z belongs to the 1st to 16th groups in the periodic table and means at least one element selected from elements other than the above Mo, Bi, Ni, Co, Fe, X, and Y; a1, b1, c1, d1, e1, f1, g1, h1, and i1 represent the number of atoms of molybdenum, bismuth, nickel, cobalt, iron, X, Y, Z, and oxygen, respectively; when $a1 = 12$, $0 < b1 \leq 7$, $0 \leq c1 \leq 10$, $0 < d1 \leq 10$, $0 < c1 + d1 \leq 20$, $0 \leq e1 \leq 5$, $0 \leq f1 \leq 2$, $0 \leq g1 \leq 3$, $0 \leq h1 \leq 5$, and i1 is a value determined by an oxidation state of each element).

4. The catalyst according to any one of claims 1 to 3, wherein a catalytically active component is carried on an inert carrier in the catalyst.

5. The catalyst according to claim 4, wherein the inert carrier is silica, alumina, or a mixture thereof.

6. The catalyst according to any one of claims 1 to 5, which is a catalyst for producing at least one of an unsaturated aldehyde compound, an unsaturated carboxylic acid compound, and a conjugated diene.

7. A method for producing at least one of an unsaturated aldehyde compound, an unsaturated carboxylic acid compound, and a conjugated diene, the method comprising using the catalyst according to any one of claims 1 to 6.

8. The method according to claim 7, wherein the unsaturated aldehyde compound is acrolein, the unsaturated carboxylic acid compound is acrylic acid, and the conjugated diene is 1,3-butadiene.

9. An unsaturated aldehyde compound, an unsaturated carboxylic acid compound, or a conjugated diene produced using the catalyst according to any one of claims 1 to 6.

**FIG. 1**

**FIG. 2**

## FIG. 3

## FIG. 4

## INTERNATIONAL SEARCH REPORT

| International application No. |
| --- |
| PCT/JP2021/000588 |

**A. CLASSIFICATION OF SUBJECT MATTER**
B01J 23/883(2006.01)i; C07B 61/00(2006.01)i; C07C 27/00(2006.01)i; C07C 47/22(2006.01)i; C07C 57/06(2006.01)i
FI: B01J23/883 Z; C07B61/00 300; C07C27/00 330; C07C47/22 A; C07C57/06
According to International Patent Classification (IPC) or to both national classification and IPC

**B. FIELDS SEARCHED**

Minimum documentation searched (classification system followed by classification symbols)
B01J23/883; C07B61/00; C07C27/00; C07C47/22; C07C57/06

| Documentation searched other than minimum documentation to the extent that such documents are included in the fields searched | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | WO 2014/051090 A1 (ASAHI KASEI CHEMICALS CORP.) 03 April 2014 (2014-04-03) claims 1-13, paragraphs [0029]-[0096], fig. 1-16 | 9<br>1-8 |
| X<br>A | JP 2014-094353 A (ASAHI KASEI CHEMICALS CORP.) 22 May 2014 (2014-05-22) claims 1-6, paragraphs [0016]-[0044], fig. 1-2 | 9<br>1-8 |
| X<br>A | WO 2017/069119 A1 (NIPPON KAYAKU CO., LTD.) 27 April 2017 (2017-04-27) claims 1-7, paragraphs [0041]-[0066], fig. 1 | 9<br>1-8 |

☐ Further documents are listed in the continuation of Box C.    ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 11 March 2021 (11.03.2021) | 23 March 2021 (23.03.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

International application no.

PCT/JP2021/000588

| Patent Documents referred in the Report | Publication Date | Patent Family | Publication Date |
|---|---|---|---|
| WO 2014/051090 A1 | 03 Apr. 2014 | US 2015/0238939 A1 claims 1-13, paragraphs [0075]-[0151], fig. 1-16 EP 2902106 A1 KR 10-2015-0046224 A CN 104661747 A | |
| JP 2014-094353 A | 22 May 2014 | (Family: none) | |
| WO 2017/069119 A1 | 27 Apr. 2017 | US 2019/0070591 A1 claims 1-7, paragraphs [0061]-[0089], fig. 1 EP 3366372 A1 CN 108136377 A KR 10-2018-0069052 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016136882 A
- JP 2017024009 A
- WO 2010038677 A
- JP 2018140326 A
- JP 2020002508 A